# EUROPEAN PATENT APPLICATION

(11) **EP 0 543 057 A1**
(43) Date of publication of application: **26.05.1993**
(21) Application number: 91203043.4
(22) Date of filing: 20.11.1991
(51) Int. Cl.: G01N 33/28

(54) **Method for determining the oil content in oil-in-water emulsions**

(71) Applicant: QUAKER CHEMICAL CORPORATION, Conshohocken, PA 19428 (US)
(72) Inventor: Broekhof, Nicolaas L.J.M., NL-1216 MB Hilversum (NL); Schellingerhout, Peter, NL-1422 MD Uithoorn (NL)
(74) Representative: de Bruijn, Leendert C.

(57) **Abstract**

The invention relates to a method for determining the oil content of oil-in-water emulsions generally containing 1-10% w/w of oil. This method is characterized by introducing an indicator in the oil phase - prior to its use in the oil-in-water emulsion - and subsequently determining the oil concentration after or during the use of the oil-in-water emulsion in a spectrophotometrical way. The indicators, suitable for use in the method according to the invention are a.o. the generally known acid/base indicators and the fluorescent indicators. The amount of indicator may vary between wide limits but lies preferably in the range of 10-500 ppm of indicator based on the oil of the oil-in-water emulsion. The data obtained with the spectrophotometrical analysis are compared with calibration curves for the system used, giving the concentration of the oil in the oil-in-water emulsion.

## Description

The invention relates to a method for determining the oil content in oil-in-water emulsions, generally having an oil content of 1-10% w/w.

### Background of the Art

Many process fluids like hydraulic fluids, metal working fluids and rolling fluids are applied as oil-in-water emulsions. The oil content of such fluids may vary between wide limits but amounts generally 1-10% w/w; see e.g. Kirk-Othmer Encyclopedia of Chemical Technology, 3. edition, Vol. 12, page 715 showing an oil content of 1-4% for High-water-base fluids usable as hydraulic fluids.

The main factor for a satisfying performance of the above-mentioned fluids is the correct oil content of these oil-in-water emulsions. Therefore, there is a need for a selective, fast, generally applicable and cheap method for determining the oil content of this type of emulsions. As generally known, under practical conditions metal working fluid emulsions and rolling oil emulsions are contaminated with e.g. hydraulic fluids, morgoils etc., which contamination is commonly indicated as "trampoil".

The methods for determining the oil content in oil-in-water emulsions known from the art are discussed in the following review.

### A) Separation of the oil phase (from the oil-in-water emulsion).

1) The oil phase may be separated from the emulsion by splitting it with for instance acids, electrolytes like AlCl₃ and other specific splitting agents. This type of determination method, however, has several drawbacks like the time-consuming aspect and the presence of trampoil, if any, which may affect the results as no distinction can be made between the "original" oil in the emulsion and the trampoil.
2) The oil phase may be separated from the emulsion by means of solvent extraction, for instance with methyl ethyl ketone as the solvent. However, this type of method is time-consuming, whereas the results may be affected by the influence of both trampoil, if present, and the loss of water-soluble components of the "original" oil in the emulsion.
3) Further the oil phase may be won by evaporating the water present in the emulsion. Besides the time-consuming aspect and the influence of the trampoil, the results of this method will be affected by the loss of volatile components in the "original" oil in the emulsion.

### B) Determination of physical parameters of the emulsion itself

1) Determination of the oil content by "TOTAL ALKALINITY". The drawbacks of this method are based on the selective depletion of certain components of the "original" oil in the emulsion and the presence of trampoil (e.g. an alkaline hydraulic fluid) which drawbacks might affect the results of the determination.
2) Determination of the oil content by "TOTAL ACID NUMBER". Next to the drawbacks of method B-1) an interference with the water hardness may arise.
3) Determination of the oil content via specific components such as
   (a) Metals; the drawbacks of this method are the necessity of expensive equipments (e.g. an atomic absorption device), the presence of trampoil and the time-consuming aspect;
   (b) Anionic emulsifiers; the drawbacks of this method are the limited applicability, the disturbance by anionic emulsifiers in trampoils as well as the presence of trampoil; and
   (c) Boric acid; the drawbacks of this method are also the limited applicability and the presence of trampoil.

Summarizing the above it may be concluded that all the methods known for the determination of the oil content in oil-in-water emulsions do have one or more of the following drawbacks.
1) The method is not specific enough: trampoil cannot be distinguished from the "original" oil in the emulsion;
2) The method is time-consuming;
3) The method is not generally applicable;
4) The method is not selective enough as selective depletion of oil components might affect the results obtained.

As apparent from the above review of possible determination methods there is still a need for a selective, fast, generally applicable and cheap method for determining the oil content of oil-in-water emulsions.

### General definition of the invention

Surprisingly it has been found that the above drawbacks may be removed by introducing an indicator in the oil phase -prior to its use in the oil-in-water emulsion- and subsequently determining the oil concentration after or during the use of the oil-in-water emulsion in a spectrophotometrical way.

### Detailed description of the invention

The term "indicator" is defined as any substance which changes from one colour to another when the hydrogen ion concentration reaches a certain value and is stable under the performance conditions of the oil-in-water emulsions.

The main types of indicators to be used in the method according to the invention are the generally known acid/base indicators and the fluorescent indicators as specified in the CRC Handbook of Chemistry and Physics, 60th edition, pages D150-D153, CRC Press Inc..

Preferably those indicators are choosen which are colourless under the performance conditions of the oil-in-water emulsions. Examples of such indicators are:
- the acid/base indicators: 2,4-dinitrophenol, ethyl red, p-nitrophenol, 6,8-dinitro-2,4-(1H) quinazolinedione, m-nitrophenol, o-cresolphthalein, phenolphthalein, ethyl-bis(2,4-dimethylphenyl)acetate, thymolphthalein, 2,4,6-trinitrotoluene and 1,3,5-trinitrotoluene, and
- the fluorescent indicators: esculin, fluorescein, dichlorofluorescein, α- and β-naphtylamine, 3,6-dioxyxanthone, brilliant diazol yellow, coumaric acid, orcinaurine, β-naphtol, and Eosine BN.

The course of the "original" oil content in the oil-in-water emulsions under performance conditions may be simply monitored by adjusting the samples of the emulsion to a proper pH-value and determining the intensity of the obtained colour of the sample spectrophotometrically and comparing the obtained result with a calibration curve for the indicator in question.

The amount of indicator to be introduced in the oil-in-water emulsions may vary between wide limits and is preferably about 10-500 ppm based on the oil component of the emulsion.

The spectrophotometer to be used in the method according to the invention may be any marketed spectrophotometer. The device used in the below-described examples is a UV/VIS-spectrophotometer manufactured by Perkin Elmer type 550S.

The conditions, under which the spectrophotometrical analysis is carried out, are standard operating conditions (see manual manufacturer).

Before the spectrophotometrical analysis may be carried out it may be necessary to make the emulsion transparent, normally by the addition of a solubilizer to the sample of the emulsion. The composition of such a solubilizer may vary, dependent on the emulsion to be treated. Examples of such solubilizers are given in the below-described Examples.

In case of used emulsions it is preferred to remove dirt particles, if present, from the emulsion by means of filtration or centrifugation before the samples are investigated spectrophotometrically. Examples of dirt particles are Fe-fines and carbon.

A further part of the invention encompasses the oil-in-water emulsions containing an indicator as defined above. Preferably the amount of indicator is 10-500 ppm, based on the oil content of the emulsion.

### LEGENDA

- Fig. 1:: This figure illustrates a graph of oil concentration versus absorption at 556.5 nm of 1%, 2% and 3% v/v emulsions of Quakerol N 1139, containing 100 ppm of phenolphthalein, in water, treated with a solubilizer defined in example 1.
- Fig. 2:: This figure illustrates a graph of oil concentration versus absorption at 509.5 nm of 1%, 2% and 3% v/v emulsions of Quakerol N 1139, containing 100 ppm of dichlorofluorescein, in water, treated with a solubilizer defined in example 1.
- Fig. 3:: This figure illustrates a graph of oil concentration versus absorption at 585 nm of 1%, 2%, 3%, 4%, 5%, 6%, 7%, 8%, 9%, 10%, 15% and 20% v/v of Quaker N 7718, containing 100 ppm of thymolphthalein, in water, added to a 3.5% v/v oil-in-water metal-working oil of Oemeta (brandname ASF 192) treated with a solubilizer defined in example 3.
- Fig. 4:: This figure illustrates a graph of oil concentration versus absorption at 585 nm of 1, 2 and 5% v/v emulsions of Quaker N 3750 ST, containing 100 ppm of thymolphthalein, in water, treated with a solubilizer defined in example 1.

### EXAMPLES

### Example 1. Rolling oil with an acid/base indicator

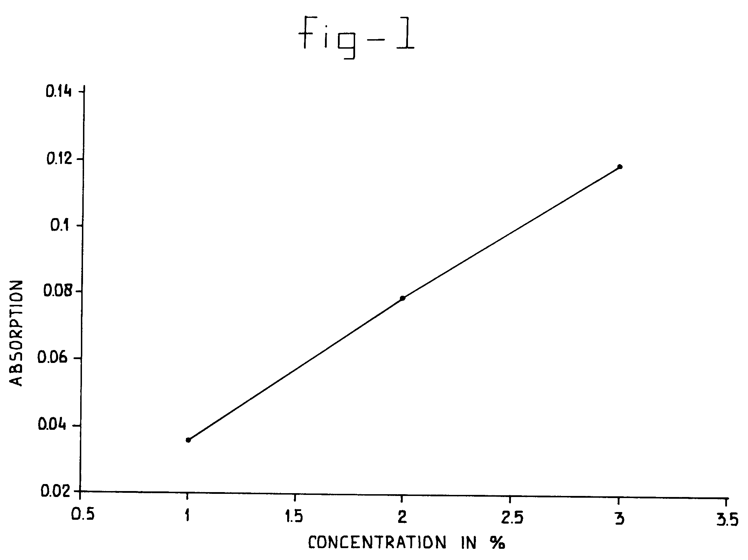

Phenolphthalein (100 ppm) was added to the commercially available Rolling oil, Quakerol N 1139. This acid/base indicator was colourless at the application pH (pH = 5-7).
A graph of concentration versus absorption was obtained by measuring the absorption at 556.5 nm of 1, 2 and 3% emulsions of Quakerol N 1139 (Fig. 1). A solubilizer was needed in order to obtain a clear solution.
The solubilizer consisted of a mixture of Tall oil fatty acids (5%), mono ethanol amine (2.5%) diethanol amine (7.5%), C12-C15 alcohol E03 (35%) and isopropylalcohol (50%).
This solubilizer (15 g) was added to 25 g of emulsion. As a result, the pH rose to 10.5 and a colour developed. The extinction at 556.5 nm was measured. In case of used emulsions dirt e.g. Fe-fines and/or carbon, was removed by filtration.

### Example 2. Rolling oil with a fluorescent indicator

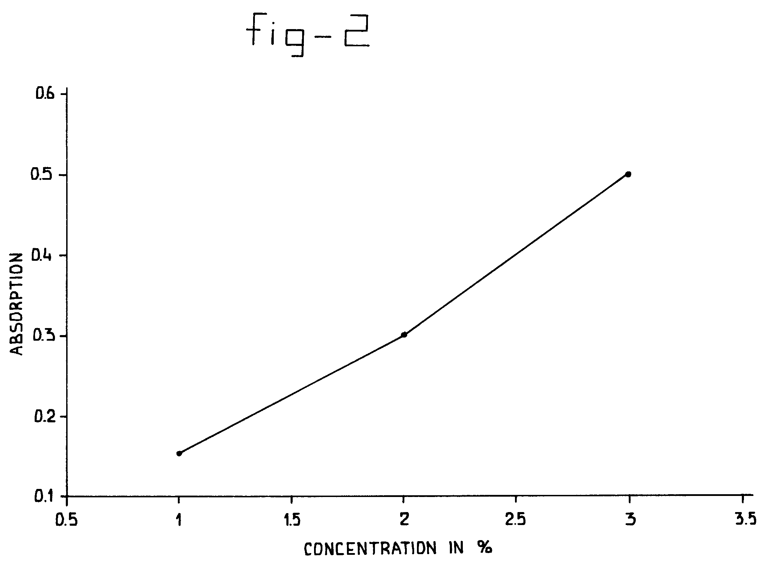

Dichlorofluorescein (100 ppm) was added to the commercially available Rolling oil, Quakerol N 1139. This fluorescent indicator was colourless at the application pH (pH = 5-7). A graph of concentration versus absorption was obtained by measuring the absorption at 509.5 nm of 1, 2 and 3% of Quakerol N 1139 (Fig. 2). The solubilizer defined in example 1 (15 g) was added to 25 g of emulsion. As a result, the pH rose to 10.5 and a colour developed. The extinction at 509.5 nm was measured. In case of used emulsions dirt e.g. Fe-fines and/or carbon, was removed by centrifugation.

### Example 3. Hydraulic fluid with acid/base indicator

Thymolphthalein (100 ppm) was added to a commercially available Hydraulic fluid, Quaker N 7718. This hydraulic fluid belongs to the Thickened High Water Content Fluids (THWCF). The aim of this example was to measure the contamination of a metal working fluid by this hydraulic fluid. A graph of concentration versus absorption was obtained by first adding 1, 2, 3, 4, 5, 6, 7, 8, 9, 10, 15 and 20% of the Quaker N 7718 to a 3.5% oil-in-water metalworking fluid of Oemeta ASF 192.
A solubilizer was needed in order to obtain a clear solution. The solubilizer consisted of a mixture of diethylene glycol monobutyl ether (100 g), sodium dodecyldiphenylsulphonate (200 g), potassium hydroxide (28.054 g) and water (till a total volume of 1 ltr).
The solubilizer (50 g) was added to each of the above-mentioned mixtures (25 g) of Quaker N 7718 and the metalworking fluid. A graph of concentration versus absorption was now obtained by measuring the absorption at 585 nm (Fig. 3).
A major advantage was the fact that this graph turned out to be almost independent of the water hardness in the range of 0-80°dH.
The fact that the Oemeta/Quaker 7718 appeared to change at 80°dH from a transparent solution to a white emulsion could easily be solved by the addition of the solubilizer, changing the emulsion again back to transparent. Disturbing dirt was removed by filtration.

### Example 4. Metalworking fluid with acid/base indicator

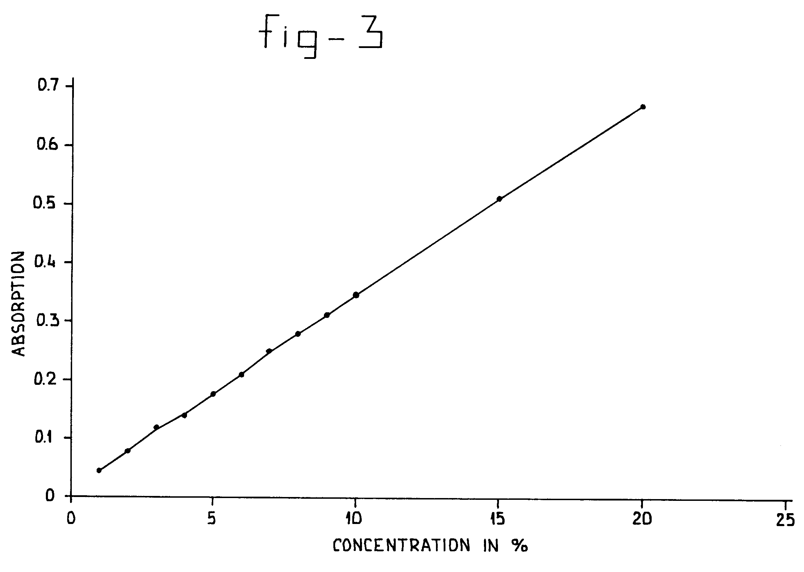

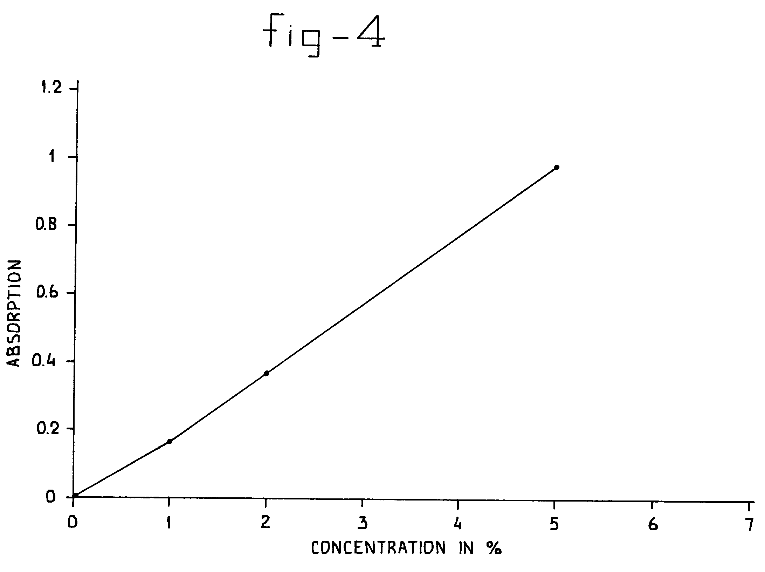

Thymolphthalein (100 ppm) was added to the commercially available Metalworking fluid, Quaker N 3750 ST.
A graph of concentration versus absorption was obtained by measuring the absorption at 585 nm of 1, 2 and 5% emulsions of Quaker N 3750 ST in alkaline water (Fig. 4).
A white Quaker N 3750 ST emulsion taken from the field could be made transparent with the solubilizer as described in example 1 (15 g was added to 25 g emulsion). Disturbing dirt was removed by filtration.

## Claims

1. A method for determining the oil content of an oil-in-water emulsion, characterized by introducing an indicator in the oil phase -prior to its use in the oil-in-water emulsion- and subsequently determining the oil concentration after or during the use of the oil-in-water emulsion in a spectrophotometrical way.

2. A method according to claim 1, characterized by using an indicator selected from the group consisting of acid/base indicators and fluorescent indicators.

3. A method according to claim 1, characterized by using an indicator, which is colourless in the pH-range of the application of the oil-in-water emulsion.

4. A method according to claims 2 or 3, characterized by using an indicator selected from the group consisting of phenolphthalein, thymolphthalein and dichlorofluorescein.

5. A method according to any of the claims 1-4, characterized by using 10-500 ppm of indicator based on the oil.

6. A method according to any of the claims 1-5, characterized by removing dirt particles from the emulsion sample prior to the spectrophotometrical analysis.

7. A method according to claim 6, characterized by removing the dirt particles from the emulsion sample by filtrating or centrifuging.

8. A method according to any of the claims 1-7, characterized by using a solubilizer for making the sample transparent prior to the spectrophotometrical analysis.

9. An oil-in-water emulsion comprising an indicator.

10. An oil-in-water emulsion according to claim 9, comprising an indicator selected from the group consisting of acid/base indicators and fluorescent indicators.

11. An oil-in-water emulsion according to claims 9 or 10, comprising an indicator selected from the group consisting of phenolphthalein, thymolphthalein and dichlorofluorescein.

12. An oil-in-water emulsion according to any of the claims 9-11 comprising an indicator in an amount of 10-500 ppm based on the oil content of the emulsion.
